# EUROPÄISCHE PATENTSCHRIFT

(11) **EP 1 172 120 B1**
(45) Veröffentlichungstag und Bekanntmachung des Hinweises auf die Patenterteilung: **09.04.2003**
(21) Anmeldenummer: 01116643.6
(22) Anmeldetag: 13.07.2001
(51) Int. Cl.: A61L 27/38, A61L 27/50

(54) **Individuelle Venenklappenprothese**
Individual venous valve prosthesis
Prothèse indiviuelle de valvule véneuse

(30) Priorität: 14.07.2000 DE 10034583
(43) Veröffentlichungstag der Anmeldung: 16.01.2002
(73) Patentinhaber: Bionethos Holding Gmbh, 31275 Immensen (DE)
(72) Erfinder: Sunnanväder, Lars, 72379 Hechingen (DE); Bader, Augustinus, Prof. Dr., 31275 Immensen (DE)
(74) Vertreter: Bösl, Raphael, Dr. rer. nat., Dipl.-Chem.

(56) Entgegenhaltungen:
- WO-A-00/32249
- WO-A-99/00152
- DE-A- 19 828 726

## Beschreibung

Die Erfindung betrifft die Verwendung einer empfängerspezifisch transformierten natürlichen Gewebematrix für die Herstellung einer individuellen Venenklappenprothese, wobei die natürliche Gewebematrix nicht azellularisiert wurde.

Der Ersatz von Klappensystemen im Körper stellt insbesonders in den kleinen Strombereichen, vor allem wenn es sich um venöse Bereiche mit geringem Volumenstrom handelt, auf Grund der Thrombosegefährdung und langen Einheilungszeiten der Implantate ein schwerwiegendes klinisches Problem dar. In der Vergangenheit hat man ohne Erfolg versucht, Venenklappen, welche mit Gerbstoffen und fixiernden Mitteln (z.B. Glutaraldehyd) entsprechend den in den großen Gefäßbereichen einsetzbaren Herzklappen behandelt wurden, zu erstellen. Man merkte jedoch, dass diese Verfahren auf Grund der hohen Thrombogenizität der Oberflächen in den kleinen Strombereichen zu einer hochgradigen Okklusionsgefährdung nach Implantation führt. Diese ist insbesonders auch durch die mangelnde Integration an den Nahtstellen evident und provoziert. Dies führte zu Überlegungen, die eine Verlängerung des Implantats beinhalteten. Das heißt, die Venenklappen und deren Ansatzstücke oberhalb und unterhalb des Klappenrings mussten proportional gesehen erheblich länger sein, als dies bei den Herzklappen schon möglich war.

So wurden Entwicklungen vorangetrieben, die Ansatzstücke von mehr als einmal des Durchmessers der Vene erforderten, oder sogar noch mehrfach länger waren. Im Grunde würde die klinische Situation jedoch einen ganz anderen Ansatz erfordern. Für therapeutische Implantate weiß man, dass nur so viel Fremdmaterial wie notwendig in der Körper eingebracht werden sollte, da dieses letztendlich einen Fremdkörper mit nachfolgenden immunologischen oder regenerativen Problemen und den damit assozierten Folgeerkrankungen bedeutet. Hierzu zählt auch die Thrombogenizität und Verkalkung des Implantats. In einer zweiten Phase wurde dann versucht, Entwicklungen die zwar schon eine verbesserte Eignung für größere Gefäße und Herzklappen zeigen auch für Venenklappen anzuwenden. Hierzu zählen Verfahren und Ansätze wie sie in DE 198 28 726 A1 offenbart wurden. Diese umfassen Azellularisierungsverfahren einer allogenen oder xenogenen Matrix unter Verwendung enzymatischer aber auch anderer Verfahren durch Einsatz oder in Verbindung mit Spülprozessen bei Gefäßen. In dieser Anmeldung waren bereits Gefäße beschrieben, wobei auch Venen unter die Definition von Gefäße fallen, was allgemein bekannt ist. Ebenso ist integraler Bestandteil des anatomischen Kenntnisstandes, dass alle Venen Venenklappen haben, denn diese sind ein physiologischer Bestandteil der Funktion der Venen. Damit soll ein gerichteter Rückfluss zum Herzen auch entgegen einer statischen Belastung ermöglicht werden. Dieses ist seit langem bekannt und Gegenstand schon sehr früher anatomischer Standardlehrwerke.

Die Anwendung der in WO 99/00152 und in DE 199 10 340 A1 offenbarten Verfahren für kleine Venen ist zwar grundsätzlich möglich, führt aber zu denselben Problemen wie bei Herzklappen mit dem Unterschied, dass diese auf Grund der Kleinheit und Fragilität der Venen hinsichtlich geringer Wandstärken, klinisch an Relevanz gewinnen und den Einsatz prohibitv werden lassen. Folgende Nachteile sind hiervon besonders klinisch relevant. Zum einen führt die starke Destabilisierung der Matrix durch Auflockerung der Faserbündel und Entfernung der Kittmolelüle wie u.a. Fibronektin und Lamin zwischen den noch verbleibenden Hauptfasern aus Collagen und Elastin und durch die enzymatischen Prozesse zu einer erheblichen Behinderung der Nähbarkeit dieser Materalien zum Zeitunkt der Implantation in den Patienten. Dies führte häufig (2/5 Fällen) zu Einrissen und Leckagen nach der Implantation. Gerade im Hinblick auf die dünnen und damit besonders vulnerablen Venenwände im klinischen Anwendungsbereich eines Beines bedeutet dies, dass hier erhebliche Thromboserisiken verbleiben. Zum anderen offenbaren die Patentanmeldungen DE 198 28 726 A1 bzw. WO 99/00152, dass es genügen würde, normale Endothelzellen und Myofibroblasten aus einer Vene des späteren Empfängers zu nehmen und damit eine Rebesiedlung mit diesen autologen Zellen außerhalb des Körpers durchzuführen, um das spätere Gefäßimplantat optimal für die Integration in den Empfängerorganismus vorzubereiten. Für diese Spendergefäßentnahmen stehen normale Gefäße wie z.B. Venen aus anderen Bereichen zur Verfügung. Niemand würde jedoch Zellen aus einer entzündeten, d.h. erkrankten Vene entnehmen, um damit eine Zellkultur in vitro anzusetzen, um eine andere neue Vene zu erstellen, da die Ursache der Entzündung z.B. in bakteriellen Prozessen liegt, die eine Kultur unmöglich machen würden. Dennoch bestehen gerade in dem klassischen Vorgehen gemäß DE 198 28 726 A1 und in den dort beschriebenen Kombinationen der verschiedenen Zelltypen Endothelzellen mit Myofibroblasten an unterschiedlichen Orten, die schwerwiegenden Nachteile, dass einerseits Zellen entsprechend der gängigen Lehre aus einem gesunden Gefäß entnommen werden und dann auf eine anderersseits azellularisierte Matrix aufgebracht werden würden. Dies bedeutet, dass Zellen mit einem ruhenden d.h. "normalen" Phänotypus, der diese nicht zu Remodelling Prozessen befähigt, entnommen werden, um danach in eine Mikroumgebung gebracht zu werden, die wiederum einen sehr statischen Zustand dieser Zellen induziert. Die Azellularisierung entfernt die Hauptinitiatoren von Remodellingprozessen, nämlich die fremden Zellen und die Zellreste, die im allgemeinen unabhängig von ihrer Herkunft die ortständigen Zellen über Kaskadenprozesse aktivieren, um von einem ruhenden in einen aktivierten Phänotypus übergehen zu können. Das heißt, dass die klassische Lehre für die notwendigen Remodellingprozesse nachteilig ist. Diese ortständigen Faktoren der Initiation von Kaskadenprozessen die multiple und genaue zeitlich und konzentrationsmäßig interagierenden Mediatorkonzentration durch z.B. chemotaktische Faktoren erfordern, lassen sich auch nicht durch externe Zugaben erfolgreich stimulieren. Dies liegt darin begründet, dass diese Faktoren zum Teil unbekannt sind oder dass die Kinetiken nicht eingehalten werden können. Zusätzlich sind derartige Faktoren teuer und würden lange Zulassungsverfahren als Arzneimittel nach sich ziehen.

Auch in WO 95/24873 werden Verfahren offenbart, die die immunologisch potentiell aktiven Zellkomponenten entfernen sollen. Diese immunogen wirkenden Komponenten sind z.B. Proteine die an Zellmembranen gebunden sind. Ebenso wird in EP 0 564 786 A2 auf verschiedene Formen der Azellularisierung eingegangen und die Lösung, die hierzu verwendet wird, beschrieben, ebenso wie Verfahren zum Einfrieren, Lagern und Rehydrieren.

In WO 00/32249 wird die Vorstellung verfolgt, dass zur Rebesiedelung von Prothesen, z.B Herzklappen, Attraktionsfaktoren verwendet werden sollen, die das Anwachsen von Endothelzellen an den Oberflächen ermöglichen sollen. Sie bezieht sich somit auf eine Prothese einschließlich eines Substrates mit einem assozierten Attraktionsstoff. Dieser soll viable Fibroblastenvorläuferzellen in vivo anziehen, z.B. auch Monozyten, und vor Ort eine Differenzierung in Fibroblasten ermöglich and das Substrat binden. Insbesonders soll diese Makrophagen spezifischen Marker (HLA-DR) besitzen, damit selektiv die richtigen Voräuferzellen für die Fibroblastengene rekrutiert werden können. Die Attraktionsfaktoren stellen natürliche Liganden oder Teile davon dar, wie z.B. CD4, HSP-70 (heat schock protein) und TCR (T-cell receptor). Ebenso werden Verfahren beschrieben, um spezifische endotheliale Vorläuferzellen anzuziehen (CD34, Flk-1, endoglin, e-Selektin, CD31). Gemäß der Offenbarung wird immer ein spezfischer Ligand verwendet, um spezifische Zellpopulationen im Sinne von Vorläuferzellen anzuziehen. Diese Liganden werden an die Matrix gebunden bevor es zur Implantation kommt. Dies führt jedoch zu einer erheblichen Reduktion des Prozesses und des Problems auf spezifische Liganden. Die Liganden können die immunologischen Stimulationsprozesse durch allogenen oder xenogenen Zelldetritus keineswegs vermeiden und im Gegenteil sogar verstärken, da es nach Implantation zu einer überfallsartigen Invasion der durch diese Liganden angezogenen Zellen wie z.B. Makrophagen kommt. Diese sollte unbedingt vermieden werden, um eine immunologische ausgelöste Zerstörung des Implantats zu vermeiden. In der Praxis sind diese keineswegs wünschenswert und ein Verfahren, das auf diese Ligandenprozesse vollständig verzichten könnte, würde eine große auch zulassungstechnische Erleichterung darstellen.

In DE 199 10340 A1 wird ein Verfahren zur Ummantelung von Venen im Herzkranzgefäßbereich beschrieben, das eine Verstärkung der Venenwand einer frisch entnommenen, vitalen Vene des Empfängers mit seinen eigenen Zellen (Fibroblasten, Muskelzellen, Endothelzellen) vorsieht, um die mechanische Belastbarkeit der Vene zu erhöhen. Hierbei handelt es sich um einen anderen Indikationsbereich am Herzen und daher nicht um eine avitale Vene, die gewollt Zelldetritus enthält; auch soll kein Remodelling induziert werden. Im Gegenteil, um ein Absterben der schon vorhanden autologen Zellen im Transplantat zu vermeinden, werden diese sofort transplantiert.

Aufgabe der vorliegenden Erfindung war daher, die oben beschriebenen Nachteile im wesentlichen zu vermeiden.

Gegenstand der vorliegenden Erfindung ist daher die Verwendung einer empfängerspezifisch transformierten mit interaktionsfähigen Zelltypen in vivo rebesiedetten natürlichen Gewebematrix für die Herstellung einer individuellen Venenklappenprothese, wobei die natürliche Gewebematrix nicht azellularisiert wurde.

Erfindungsgemäß wird somit ein sich von der klassischen Lehre abhebendes völlig anderes und entgegengesetztes Verfahren eingesetzt. Hierzu wird eine native Matrix unter Vermeidung von Azellularisierungsprozessen und unter Vermeidung von Attraktionsligandzugängen mit den interaktionsfähigen Zelltypen in vitro rebesiedelt und dadurch zu spezifischen Interaktion stimuliert.

Diese vorzugsweise empfängerverträglichen und insbesondere autologen Zellen beinhalten als Basis vor allem Fibroblasten, Muskelzellen und Endothelzellen. Myofibroblasten werden im allgemeinen bereits duch Zelldetritus initiiert und führen einen Aktivierungsswitch durch. Besonders verstärkt wird dieser noch zusätzlich durch die Präsenz von durch Zellreste oder Partikel aktivierte Makrophagen, die dadurch für Remodellingprozesse spezifische Aktivierungskocktails freisetzen. Diese sind zu unterscheiden von immunologischen Aktivierungskocktails, die in vivo freigesetzt würden, solange die fremden Zellen allogenen oder xenogenen Ursprungs in einem Organismus in vivo eingebracht werden würden. Erfindungsgemäß findet jedoch ein selektiver, d.h. partikelinduzierter Aktivierungsprozess außerhalb des Körpers in vitro z.B. in Bioreaktoren statt. Es kann dort vorteilhafterweise nicht zu Leukozyten und Plasmazellenaktivierungen kommen.

Da die Aktivierungsprozesse im allgemeinen zwei Komponenten haben, einerseits durch die Art- und Zusammensetzung der Matrix als auch durch die Art und Präsenz der Aktivierungszellen, sind erfindungsgemäß die Aktivierungsprozesse im wesentlichen unterschiedlich und willkürlich steuerbar.

Die endogene Partikelinduktion durch in der Matrix des späteren Implantats noch initial gelegene Zellreste (Detritus) kann somit teilweise oder ergänzend exogen durch Zugabe von Partikeln erreicht weden. Diese sind vorzugsweise kleiner als ca. 15µm und werden Makrophagenkulturen hinzugesetzt, von denen dann die Überstände verwendet werden um die Myofibroblasten auf der Matrix aktivieren zu könnnen.

Unter einer "empfängerspezifischen Transformation" wird dabei vorzugsweise eine Besiedlung der ausgewählten Matrix mit empfängerverträglichen Zellen, insbesondere mit autologen Zellen des Protheseempfängers verstanden und zwar auf der Basis der oben beschriebenen erfindungsgemäßen Verfahren unter Integration der Aktivierungsprozesse.

Die unten noch näher beschriebene Matrix wird soweit mit empfängerverträglichen Zellen besiedelt, dass die Thrombogenizität des Fremdkörpers "Venenklappenprothese" hinreichend unterdrückt wird. Auch die Art der aufgesiedelten Zellen kann auf die Thrombogenizität einen Einfluss haben. Besonders geeignet ist eine alleinige Besiedlung mit Fibroblasten und Endothelzellen sowie auch ggf. mit Myofibroblasten.

Die Matrix, die für die empfängerspezifische Transformation verwendet wird, kann auch eine für den Empfänger xenogene oder allogene Matrix sein. In diesem Fall können exogene Aktivierungsprozesse durch phagozytierende Makrophagen kombiniert werden.

Vorzugsweise kann die für die empfängerspezifische Transformation verwendete Grundmatrix eine natürliche Venenklappe oder Arterienklappe sein.

Besonders vorteilhaft ist es, wenn die Matrix, insbesondere die Oberfläche der Matrix, Kleinstpartikel enthält, die vorzugsweise phagozytierbare oder degradierbare Materialien enthalten. Beispielsweise kann die Oberfläche der Matrix vollständig beschichtet sein oder der Anteil von Kleinstpartikel zu Matrix liegt bei ca. 15-60% bzw. ca. 15-85%. Als Materialien sind beispielsweise Polylaktide, Polyester, Polyurethane, Polyhydroxyalkanoate, Stärke- oder Zuckerderivate, Lipide, Proteine oder Kollagene, vorzugsweise Polylaktide geeignet. Der Durchmesser der Partikel liegt vorzugsweise bei bis zu ca. 15 Mikrometer (µm). Besonders geeignet sind Kleinstpartikel, die eine verlängerte Halbwertszeit von beispielsweise 2 Wochen besitzen. Derartige Kleinstpartikel, z. B. Polylaktidpartikel, sind allgemein erhältlich.

In alternativer Verfahrensweise kann ein empfängerspezifisch transformiertes Matrixmaterial für die Konstruktion einer Venenklappenprothese verwendet werden. Hierbei kann so vorgegangen werden, dass die Venenklappenprothese aus Matrixmaterialien zusammengesetzt ist. Die ausgewählten Materialien werden vor der Konstruktion der Venenklappenprothese vorbesiedelt und können ggf. nach der Konstruktion in einem weiteren Verfahrensschritt mit einem durchaus auch thrombogenen Material wie z.B. extrazellulärer Matrix (Collagenen, Fibrin) an der Oberfläche beschichtet und zusätzlich weiter mit empfängerspezifischen Zellen besiedelt werden.

Die Venenklappenprothese umfasst dabei vorzugsweise wenigstens ein Klappensegel.

Um einen besseren Anschluss an die Vene des Empfängers zu gewährleisten, kann die Venenklappeprothese in bevorzugter Ausbildungsform ein Venenstück einer gewissen Gesamtlänge umfassen, wobei vorzugsweise vorgesehen ist, dass die Venenklappe sich in einem Venenstück befindet, dessen Länge oberhalb und unterhalb des Klappenbereiches auch in vorteilhafter Weise weniger als einmal dem Durchmesser der Vene entsprechen kann.

Ein großer Vorteil der Erfindung liegt darin, dass die in der angegebenen Weise prozessierte und ggf. für den Empfänger speziell neu konstruierte Venenklappe in einem Verfahrensschritten so vorbereitet werden kann, dass die Assimilation mit dem Empfänger durch das bereits in vitro einsetzende verstärkte Remodelling im wesentlichen vollständig ist. Dies beinhaltet auch die Integration und optimale Erzeugung einer Kontinuität an den Nahtstellen als den Orten, die sonst die maximale Thrombosegefährdung bedeuten. An diesen Orten müssen selbst geringe Gefährdungen ausgeschlossen werden, da mininmale Thrombosen zu sofortigem Implantatversagen führen würden und Langzeitschäden wie venöse Insuffizienz, nutrititive Störungen und Wundheilungsstörungen hervorrufen würden.

Vorteilhaft ist auch, dass die Funktion der Venenklappenprothese wenigstens in vitro getestet werden kann, indem die Venenklappe in eine entsprechende Vorrichtung eingespannt und pulsierend mit einem Kulturmedium oder auch einfacher (kristalloider) Lösung perfundiert wird.

### Ausführungsbeispiele:

### Beispiel 1

Ein allogene Vene wurde nach der Entnahme in eine Bioreaktorenvorrichtung eingebracht und dort fixiert. Autologe Zellen bezüglich des späteren Implantatempfängers wurden in Kulturflaschen unter Beachtung üblicher steriltechnischern Kautelen und Standardmedien für 2 Wochen expandiert. Diese Zellen wurden nach Ablösung von den zur Expansion verwendeten Kulturoberflächen im Falle von Endothelzellen luminal und an der Peripherie im Falle von Myofibroblasten einer allogenen Vene aufgebracht. Hierfür wurden Standardkulturmedien verwendet. Diese Zellen ließ man dort für ca. 1-2 Wochen weiterwachsen und in die Tiefe integrieren. Während dieser Phase wurde eine Perfusion der Vene mit Kulturmedium, das alle 2 Tage erneuert wurde, kontinuierlich vorgenommen. Für den Besiedlungsprozess kann kryokonserviertes oder zusätzlich sterilisiertes Gewebe verwendet werden.

Falls der Venenklappenumbauprozess weitergehend beschleunigt werden soll, so kann die gesamte aus ca. 10 ml Blut über Standardisolationsverfahren des Patienten erhaltene Makrophagenpopulation an den Innensseiten der Bioreaktoren, d.h. räumlich getrennt von dem zukünftigen Implantat aufgebracht werden. Diese adhärieren dort ohne weiteres auch auf Kunststoffoberflächen, Membranen oder Glas. Durch die während der Perfusion der Vene freigelösten Partikel - und Zellwandbestandteile der ursprünglichen allogenen Zellen kommt es zu Aktivierung der Makrophagen. Diese sezernieren direkt in das in den Bioreaktoren zirkulierende Kulturmedium Cytokine und Wachstumsfaktoren. Die stimuliert freigegebenen Cytokine werden in makrophagenspezifischen Kinetiken und Mengen vor Ort freigegeben. Dieser Faktorenkocktail, der nicht weiter beeinflusst werden soll, wirkt wiederum chemotaktisch und migrationsinduzierend auf die glatten Muskelzellen und Fibroblasten, die an der Peripherie der Vene als auch luminal aufgebracht werden können. Es kommt dadurch zu gerichteten Wanderungs- und Remodellingprozessen, da die Myofibroblasten einen phänotypischen Switch in den sog. sekretorischen Zustand durchführen. Destruktrionsprozesse durch die Makrophagen werden vermieden, da diese keinen physischen Kontakt zum Implantat erhalten könnnen.

### Beispiel 2

Eine natürliche Gewebematrix, die in den Oberflächen der Gewebefasern auch einen Anteil von beispielsweise 15% von Polylaktidpartikeln mit einem Durchmesser von z. B. ca.14 um mit verlängerter Halbwertszeit enthält und die sich vorzugsweise ca. zwei Wochen länger hält als die kurzfristig sich auflösenden Oberflächenstrukturen aus Polylaktid, wird mit Myofibroblasten beidseits unter Verwendung konventioneller Nährmedien besiedelt. Durch die Perfusionsprozesse in den Bioreaktoren und das Einwirken der wässrigen Lösungen werden Partikel aus den oberflächlichen Strukturen herausgelöst, ohne die Gesamtstabilität zu beeinträchtigen. Diese Partikel werden von den Myofibroblasten phagozytiert und zu den fakultativ angebrachten Makrophagen an den Innenwänden der Bioreaktoren über das Medium herangeführt und von diesen ebenso phagozytiert. Dies führt zu den gerichteten phänotypischen Veränderungen der Myofibroblasten und einer verstärkten lokalen Remodellingaktivität.

5 Tage nach Beginn der Myofibroblastenbesiedlung werden Endothelzellen auf die dann bereits konfluenten und in die Tiefe vorgedrungenen Myofibroblasten aufgegeben und 2 weitere Tage kultiviert. Danach sind die Außenstrukturen bereits durch eine autologe Matrix ersetzt und eine interne Kernstruktur mit variabel einstellbarer Halbwertszeit ist noch erhalten.

Das Implantat kann zu diesem Zeitpunkt bereits implantiert werden. Die Umbauprozesse werden in vivo zu Ende geführt. Die Makrophagen-aktivierenden Partikel sind zu diesem Zeitpunkt bereits durch die phagozytierenden Zellen entfernt worden.

## Patentansprüche

1. Verwendung einer empfängerspezifisch transformierten mit interaktionsfähigen Zelltypen in vitro rebesiedelten natürlichen Gewebematrix für die Herstellung einer individuellen Venenklappenprothese, **dadurch gekennzeichnet, daß** die natürliche Gewebematrix nicht azellularisiert wurde.

2. Verwendung nach Anspruch 1, **dadurch gekennzeichnet, dass** die empfängerspezifische Transformation eine Besiedlung der Matrix mit empfängerverträglichen Zellen, vorzugsweise autologen Zellen des Prothesenempfängers, umfasst.

3. Verwendung nach Anspruch 1 oder 2, **dadurch gekennzeichnet, dass** die Matrix eine xenogene oder allogene Matrix ist.

4. Verwendung nach einem der Ansprüche 1-3, **dadurch gekennzeichnet, dass** die Matrix eine natürliche Venenklappe oder Arterienklappe ist.

5. Verwendung nach einem der Ansprüche 1-4, **dadurch gekennzeichnet, dass** die Matrix Kleinstpartikel, vorzugsweise phagozytierbare oder degradierbare Kleinstpartikel, enthält.

6. Verwendung nach einem der Ansprüche 1-5, **dadurch gekennzeichnet, dass** die Venenklappenprothese aus empfängerspezifisch transformiertem Matrixmaterial konstruiert wird.

7. Verwendung nach einem der Ansprüche 1-6, **dadurch gekennzeichnet, dass** die Venenklappenprothese wenigstens ein Klappensegel umfasst.

## Claims

1. Use of a recipient-specific transformed natural tissue matrix which is recolonized in vitro with cell types which are able to interact for the production of an individual prosthesis for a venous valve, **characterized in that** the natural tissue matrix was not acellularized.

2. Use according to claim 1, **characterized in that** the recipient-specific transformation comprises a colonization of the matrix with recipient-compatible cells, preferably autologous cells of the recipient of the prosthesis.

3. Use according to claim 1 or 2, **characterized in that** the matrix is a xenogene or allogene matrix.

4. Use according to any of the claims 1-3, **characterized in that** the matrix is a natural venous valve or an arterie valve.

5. Use according to any of the claims 1-4, **characterized in that** the matrix contains smallest particles, preferably phagocytable or degradable smallest particles.

6. Use according to any of the claims 1-5, **characterized in that** the prosthesis for venous valve is constructed of recipient-specific transformed matrix material.

7. Use according to any of the claims 1-6, **characterized in that** the prosthesis for venous valve comprises at least one valvular cusp.

## Revendications

1. Utilisation d'une matrice tissulaire naturelle transformée d'une manière spécifique au receveur et repeuplée in vitro avec des types de cellules capables d'interactions pour la fabrication d'une prothèse individuelle de valvule de veine, **caractérisée en ce que** la matrice tissulaire n'a pas été rendue acellulaire.

2. Utilisation selon la revendication 1, **caractérisée en ce que** la transformation spécifique au receveur comprend le peuplement de la matrice avec des cellules compatibles avec le receveur, de préférence avec des cellules autologues du receveur de la prothèse.

3. Utilisation selon la revendication 1 ou 2, **caractérisée en ce que** la matrice est une matrice xénogénique ou allogénique.

4. Utilisation selon l'une des revendications 1 à 3, **caractérisée en ce que** la matrice est une valvule de veine ou une valvule artérielle naturelle.

5. Utilisation selon l'une des revendications 1 à 4, **caractérisée en ce que** la matrice contient des microparticules, de préférence des microparticules pouvant être phagocytées ou dégradées.

6. Utilisation selon l'une des revendications 1 à 5, **caractérisée en ce que** la prothèse de valvule de veine est constituée d'une matière matricielle transformée d'une manière spécifique au receveur.

7. Utilisation selon l'une des revendications 1 à 6, **caractérisée en ce que** la prothèse de valvule de veine comprend au moins une voilure de valvule.
